Europäisches Patentamt

European Patent Office

Office européen des brevets

(11) Publication number: **0 042 240**
**A1**

## EUROPEAN PATENT APPLICATION

(21) Application number: 81302515.2

(22) Date of filing: 05.06.81

(51) Int. Cl.³: **A 61 B 3/14,** A 61 B 3/00,
A 61 B 3/02

(30) Priority: 16.06.80 GB 8019645

(43) Date of publication of application: 23.12.81
Bulletin 81/51

(84) Designated Contracting States: DE FR

(71) Applicant: Atkinson, Janette, 67 Barton Road,
Cambridge (GB)
Applicant: Braddick, Oliver John, 67 Barton Road,
Cambridge (GB)
Applicant: Howland, Howard Chase, 205 Winston Drive,
Ithaca New York (US)

(72) Inventor: Atkinson, Janette, 67 Barton Road, Cambridge
(GB)
Inventor: Braddick, Oliver John, 67 Barton Road,
Cambridge (GB)
Inventor: Howland, Howard Chase, 205 Winston Drive,
Ithaca New York (US)

(74) Representative: Irish, Vivien, Elizabeth, Patent
Department National Research Development
Corporation P.O. Box 236 Kingsgate
House 66-74 Victoria Street, London SW1E 6SL (GB)

(54) Simplified refractometer.

(57) A method of testing an eye for defocus comprises illuminating the retina with a flash of light from a point source (30); at a position surrounding the point source (30) receiving light reflected by the retina and recording the received light through a radially symmetrical focusing system (22) focused closer than the eye; repeating the illumination and recording the received light through a radially symmetrical focusing system (22) focused beyond the eye; and from the two records determining the direction of any defocus of the eye.

- 1 -

120341

## SIMPLIFIED REFRACTOMETER

This invention relates to refractometers, that is instruments for the ophthalmic testing of humans, animals or birds.

In US Patent No. 3,879,113, H.C. Howland and B. Howland described an attachment for a camera which can be used to illuminate and photograph a test subject in such a way as to allow estimations of the refractive states of both eyes simultaneously, even with a non-cooperative subject such as a very young child. An application of the device is described in Vision Research, Volume 19, 1979, pages 1319 to 1330 in a paper by O. Braddick et al. Such a refractometer may be referred to as a "radially unsymmetrical refractometer" because the use of cylindrical lenses introduces directional effects into the recorded light.

A disadvantage of the method is that the direction of any refractive error, i.e. myopic or hypermetropic, cannot be readily determined, and that the axis of any astigmatism cannot be directly determined from a single photograph.

The present invention relates to a simplified form of the known device.

According to the invention, a photorefractometer comprises a variable focus optical recording means containing a radially symmetrical focusing system; a hollow cylinder of diameter at least equal to the aperture of the recording means arranged close to and coaxially with the focusing system and containing only an optical fibre light guide arranged with one end coaxial with the cylinder and directed away from the recording means; and a flash source of light to which the other end of the optical fibre is connected.

The use of a radially symmetrical focusing system eliminates the directional effect introduced by the recording apparatus in the prior art arrangement. The instrument refracts light equally in all meridians of its optical system; any directional effect is introduced by defects in the eye under test.

The optical recording means may be a photographic camera containing one or more spherical lenses.

Also according to the invention, an attachment for use in photorefractometry comprises a hollow cylinder; means to attach the cylinder to the front of a variable focus optical recording means containing a radially symmetrical focusing system, the cylinder having an internal diameter at least equal to the aperture of the camera, being attached coaxially with the focusing system, and containing only an optical fibre arranged with one end coaxial with the cylinder and directed away from the recording means; and means to connect the other end of the fibre to a flash source of light.

Further according to the invention, a method of testing an eye for defocus comprises illuminating the retina with a flash of light from a point source; at a position surrounding the point source receiving light reflected by the retina and recording the received light through a radially symmetrical focusing system focused closer than the eye; repeating the illumination and recording the received light through a radially symmetrical focusing system focused beyond the eye; and from the two records determining the direction of any defocus of the eye.

The axial orientation of any astigmatism of the eye can also be easily seen and determined on either of the records.

The method of testing may further comprise repeating the illumination and recording the received light through a radially symmetrical focusing system focused at the eye; and from the three records determining the magnitude of any defocus of the eye.

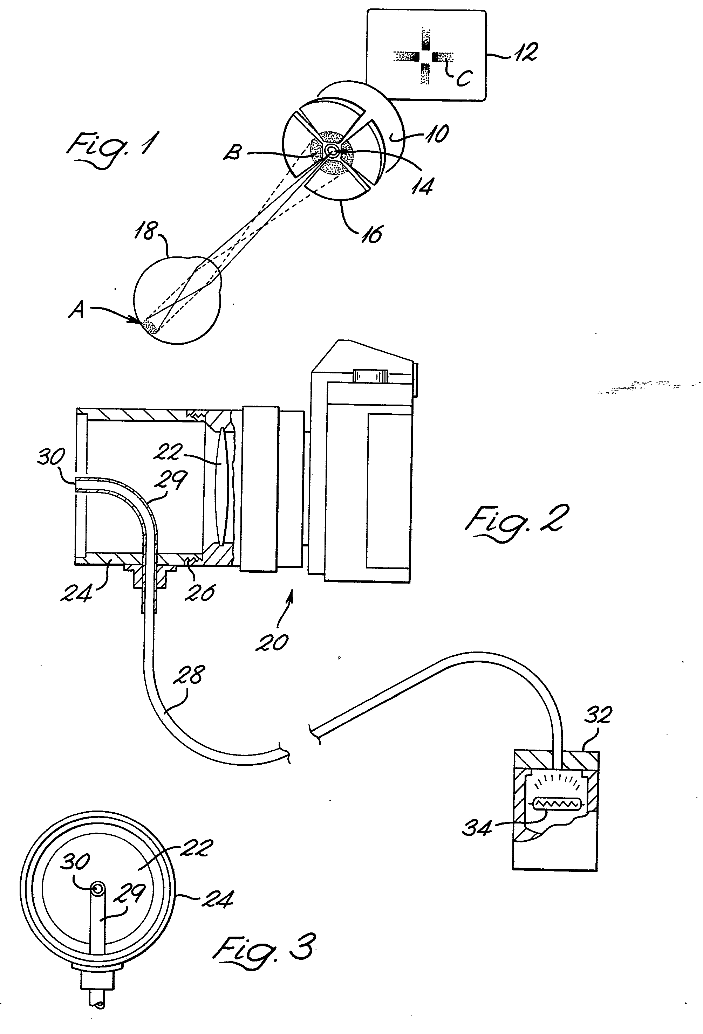

In the accompanying drawings, Figure 1 is a view of the optical arrangement of the aforementioned prior art photorefractometer described by Howland and Howland. The invention will be described by way of example with references to:-

Figure 2, which is a sectional side view of the photorefractometer system according to the invention;

Figure 3, which is a front view of the photorefractometer attachment shown in Figure 2;

- 3 -

Figures 4, 5, 6 and 7 are ray diagrams showing the path of light in the system for four settings of the eye focus when the camera focus is fixed; and

Figures 8 and 9, which are plots of radius of reflected light spot against distance at which the eye is focused for two different focal lengths of the camera.

In Figure 1, in the prior art arrangement, a camera lens 10 is positioned in front of a film plane 12 in a conventional but large aperture camera which is focused at the distance of the test subject. In front of the lens, placed centrally and facing away from the camera is the end 14 of an optical fibre, the other end of which is connected to a flash source of light (not shown). Spaced around the end 14 of the fibre and in the same plane are four cylin-drical lens segments 16 which alter the focus of the camera. These segments and the fibre are carried by an attachment to the camera (not shown). An eye under test is shown, reference 18.

In use, the eye (in fact both eyes) is directed towards the camera, is illuminated with a flash of light, as indicated by the arrowed full lines, and light is reflected by the retina, as shown by the broken lines, towards the camera which is operated simultaneously with the flash to record light reflected by the subject's eyes.

If the received light is perfectly focused on the retina by the lens in the eye, the rays reflected by the retina will fall on the end of the optical fibre and will not reach the film plane 12. If, however, the eye is myopic (as shown), then the light covers an area A of the retina, shown dotted, and reflected light will fall on an area B (also shown dotted) of the cylindrical lens segments and will be focused on the film at film plane 12 as a cross-shaped pattern C by the cylindrical lens segments and the camera lens acting in combination. The lengths of the arms of the pattern are directly proportional to the degree of defocus in the corresponding meridian of the eye. The cross pattern is superimposed on a photograph of the subject which is highly degraded by the four cylindrical lenses.

- 4 -

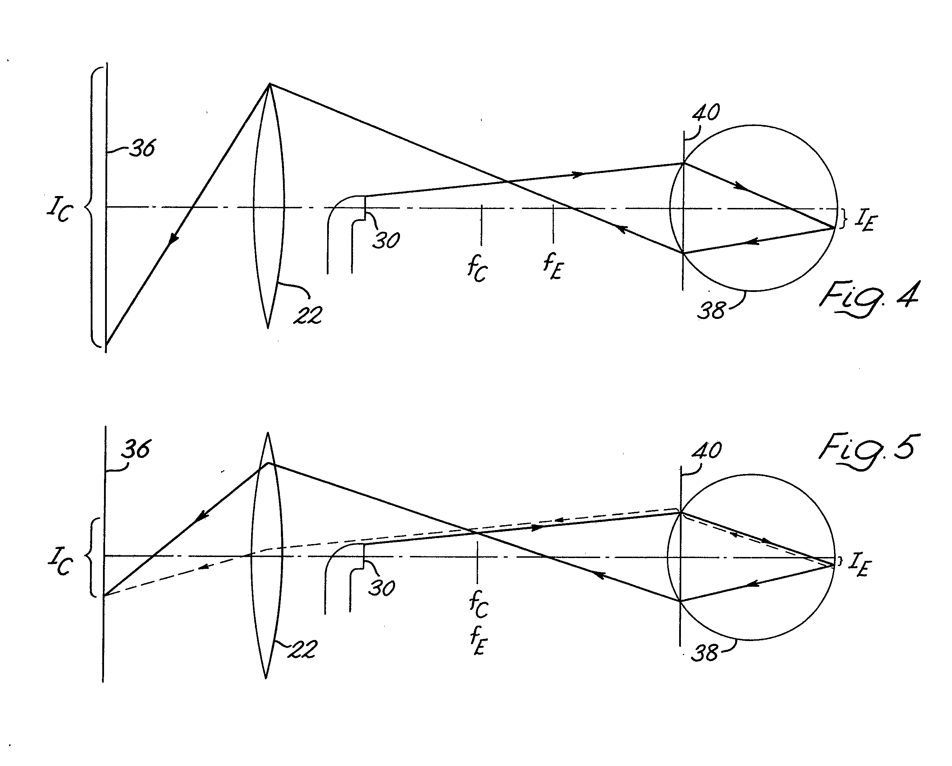

In the apparatus according to the present invention, the cylindrical lens segments have been omitted to allow use of a test method of greater scope than in the prior art. As shown in Figures 2 and 3 in which an attachment to a camera 20, the camera having a lens 22, consists of a tube 24 of a diameter slightly larger than the aperture of the camera and is attached to the camera coaxially with the lens 22 by a screw fitting 26. The tube 24 carries one end of an optical fibre 28 in a rigid guide 29, the end face 30 being in the centre of the tube (and of the camera lens) and directed away from the camera towards a test subject. The other end of the fibre is connected to a sealed unit 32 containing a flash source 34.

If the subject is illuminated by a flash of light, instead of a cross-shaped pattern as in Figure 1, the camera film records a blurred circle of light superimposed on a uniformly blurred, but recognisable, photograph of the test subject. The present invention is based on the surprising discovery that this optically simple arrangement provides considerably more information than the optically complex prior art device. The formation of the light circles is illustrated in the ray diagrams of Figures 4, 5, 6 and 7, which show the extreme rays limiting the image diameters for four different positions of focus of the eye.

Figure 4 shows the endface 30 of the optical fibre, the camera lens 22 and the camera film plane 36 with the camera focused at $f_c$ closer than the subject. Suppose the eye 38 is focused at $f_E$, closer to the eye than focus $f_c$. Light from the fibre diverges towards the eye 38; the ray entering the extreme edge of the eye pupil in the pupil plane 40 is shown. In the eye, an image $I_E$ is formed on the retina; the image is blurred, and its diameter is shown. Light in this ray which is reflected by the retina through the other extreme edge of the pupil determines the size of the image $I_c$ on the film in the camera.

Figure 5 shows the situation when the eye is focused at the same point as the camera. Again, the extreme ray accepted by the pupil is shown. In this case the ray which leaves the same side of the pupil as it enters and the ray which leaves the opposite side of the pupil would coincide at the film plane 36, but the ray leaving the same side of the pupil as it enters is in fact obstructed by the endface 30 of the fibre, so the size of the image at the film plane is determined by the other ray; the obstructed ray is shown by a broken line.

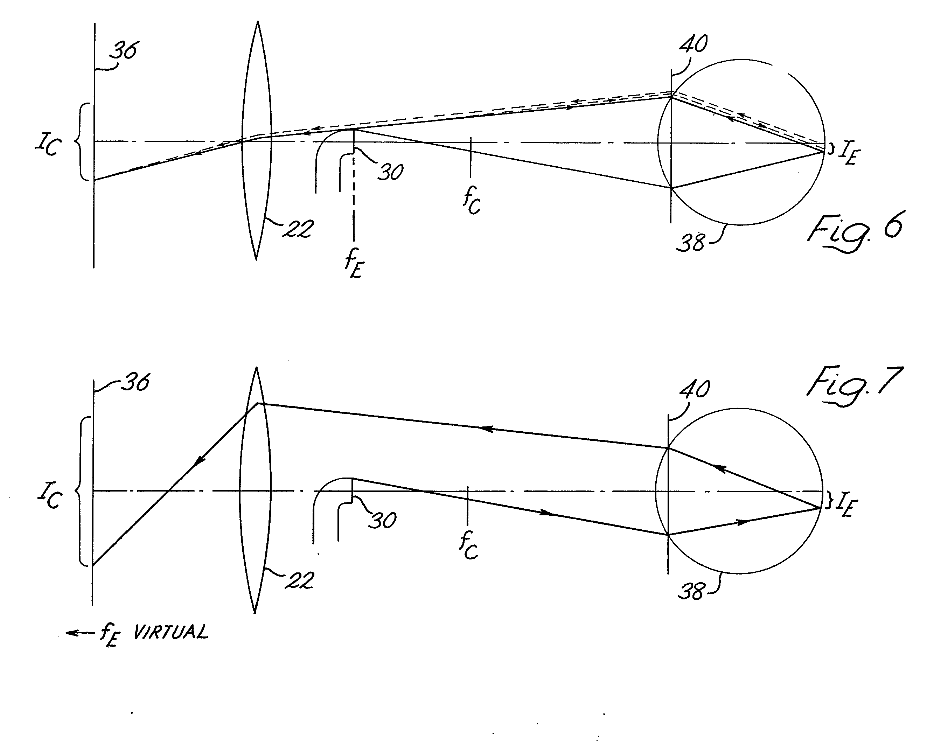

In Figure 6, the eye is focused at $f_E$ on the endface 30 of the optical fibre, and the camera is focused at $f_c$ as before. A ray entering and leaving the pupil at the same extreme edge, and a ray entering and leaving at opposite edges again coincide to form the extra ray which limits the size of an image $I_c$ on the film in plane 36.

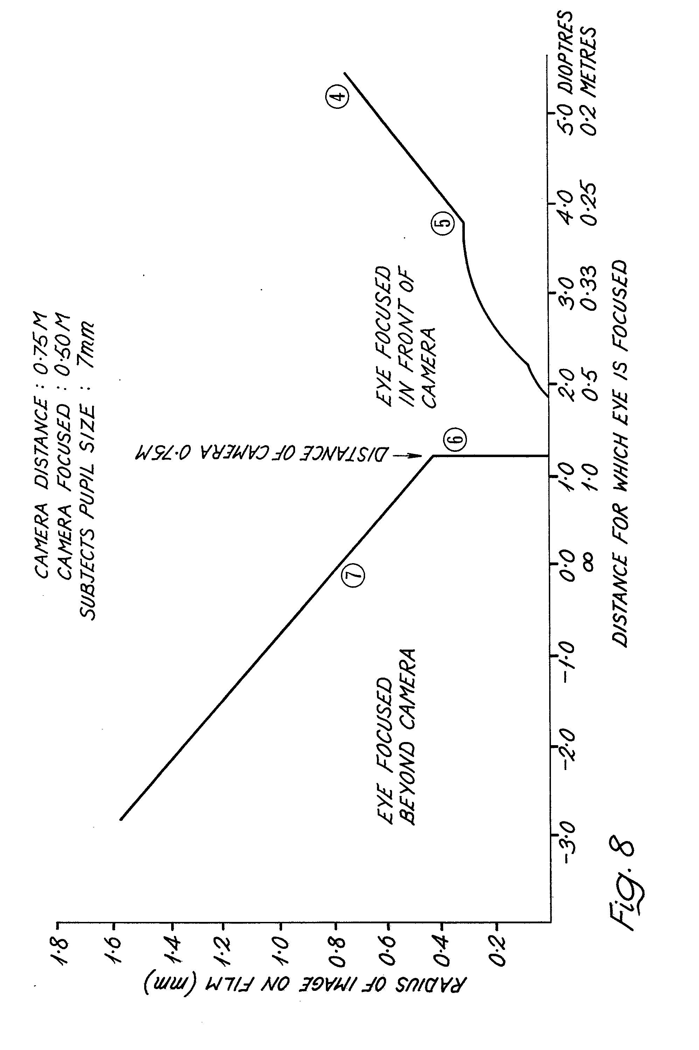

In Figure 7, the eye 38 is focused beyond the camera, and the camera is focused at $f_c$. The extreme ray which forms an image enters the pupil of the eye 38 at one edge and leaves by the opposite edge.

In all of Figures 4, 5, 6 and 7, the image formed on the film plane is a blurred circle, the diameter varying with the position at which the eye is focused. The blurred circle is an image of the pupil of the eye. In Figures 4 and 7, as the relative defocus of the eye increases, the extreme ray enters the camera at an increased angle to the axis so that the diameter of the circle increases.

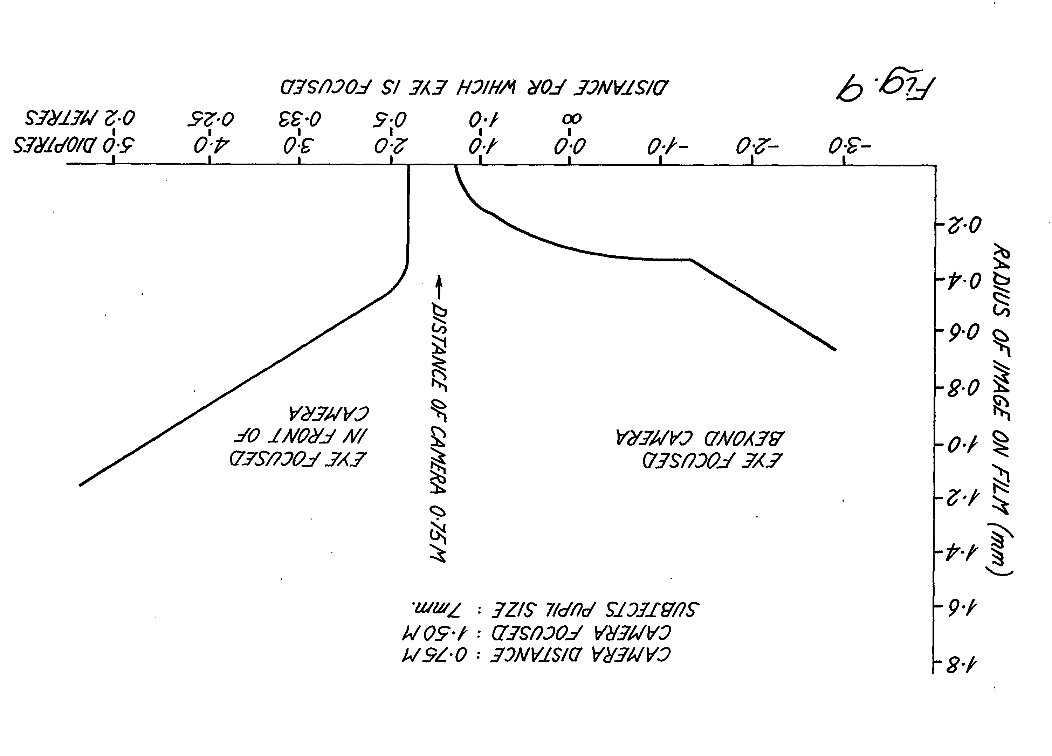

Using information of the type illustrated in Figures 4 to 7, a computer plot was derived showing the radius of the blurred bright image of the eye pupil recorded on the film against the distance for which the eye is focused. In Figures 8 and 9 the distance of the camera from the eye was 0.75 metres and the subject's pupil size was 7 millimetres. In Figure 8 the camera is focused at a distance of 0.50 metres, i.e. in front of the eye, and in Figure 9 the camera is focused at a distance of 1.50 metres, i.e. behind the eye of the subject. In both cases, the image of the pupil is of smallest radius when the eye is focused on the light source. Over a considerable range of focal distance of the eye, the radius of the image formed on the film increases linearly with the eye's defocus in dioptres relative to the position of the camera. The four conditions illustrated in Figures 4 to 7 are referenced on Figure 8.

In both Figures 8 and 9 the variation is not symmetrical; in Figure 8, there is a region where the image radius is small when the eye is focused in front of the camera, and in Figure 9 the image is small when the eye is focused beyond the camera. [The irregularities in the functions are due to obstruction of some rays by the fibre guide 29 and endface 30.] In the method of the present invention therefore, records are made of the pupil images with the camera focused respectively in front of and behind the eye; examination and comparison of the photographs then allows an assessment not only of the degree of defocus of the eye, but also its direction, i.e. long- or short-sighted. It is a great advantage that this assessment can be made without the need for full co-operation on the part of the subject. So long as the eye is directed towards the camera to within about $5^o$, an estimation of short- or long-sight can be made. This allows the method to be applied to very young children and to animals and birds by attracting their attention to the region of the camera.

Reference has been made so far to the photographic record of a blurred circle due to a focusing defect. It is another great advantage of the present method that any astigmatism is immediately obvious because a blurred ellipse is then recorded, and the axis of the ellipse, and therefore of the defect, can be accurately determined from a single photograph.

To give a specific example, referring to Figures 8 and 9, for a myopic eye, focused in front of the camera, the blurred circle will be of larger diameter when the camera is focused at 1.5 metres than when it is focused at 0.5 metres. For a long-sighted eye the relative diameters of the blurred circles will be reversed.

- 7 -

Further, a measurement of the actual diameters of the circles, plus a measurement of pupil size from a fully focused photograph of the subject and use of the scales in Figures 8 and 9 or analogous charts computed for the particular pupil size allows an estimation of the magnitude of the defocus of the eye.

Since the circles are blurred, a judgement will always have to be made to determine their size.

When astigmatism is present, and blurred ellipses are recorded, meridia in the eye corresponding to the long and short axes are considered separately.

It is an essential feature that a camera is used which has a large aperture - the aperture determines the maximum refractive error of the eye which can be sensed. If the diameter of the bundle of returning rays at the plane of the fibre endface is larger than the camera aperture, then information is lost and "vignetting" occurs. A typical camera may have a 55 millimetre focal lens of $f = 1.2$, which is equivalent to a camera aperture of 46 millimetres.

It is also preferable to have the endface of the optical fibre as close as possible to the camera film plane, since a child whose attention is attracted will probably focus on the camera operator's face rather than the fibre endface, but this distance is usually short and can be accommodated.

It is expected that a major application of the apparatus according to the invention will be in the screening of young children to detect defects of vision. This may be carried out by unskilled personnel who merely need to measure the size of the circle of light on several photographic records of the subject taken with different, known, camera settings and compare them with predetermined limits, and also to look for indications such as ring formations in the blurred circle which indicate myopia. After such a screening process, further investigation of any defect can be made, either using the prior art Howland device described above (which cannot easily be used to determine the sign of the defect) or by retinoscopy; skilled operators are required in both cases.

A further advantage of use of the simplified device according to the invention is that, if the camera is focused on the patient's eyes, the size of the brightly lit pupil can be directly measured from the photograph, knowing the other variables. Such a measure is needed for quantitative interpretation because as pupil size increases, the diameter of the blurred circle increases for a constant error of the eye focus. Using the Howland device, pupil size had to be obtained by an additional measurement using separate equipment. Now it can be obtained from a third photograph taken with the other two records.

Yet another advantage is that the photographic record also shows the corneal reflection of the flash, as a circle of different intensity within the image of the pupil obtained by retinal reflection. Any squint in the patient is clearly apparent and, again, it is advantageous that co-operation is not required. Also, any malformation of the pupil or opacity of the lens is clearly recorded, and screening for such defects can be undertaken simultaneously with the screening for long- or short sight.

Further advantages are that several indicators of malfunction are available to unskilled operators. For example a circle with a clear edge instead of a blurred edge indicates the effect of vignetting - the error in the eye causes a divergence of the light which is greater than can be accepted by the camera. Also in some situations the shadow of fibre guide 29 is recorded, and its presence or absence indicates the degree of defocus of the eye.

The provision of a blurred but recognisable record of the subject also allows easy identification of the subject.

In a variation, the camera 20 is a video camera in a video or other image recording system.

0042240

- 9 -

CLAIMS

1. A photorefractometer comprises variable focus optical recording means containing a radially symmetrical focusing system; a hollow cylinder of diameter at least equal to the aperture of the recording means arranged close to and coaxially with the focusing system and containing an optical fibre arranged with one end coaxial with the cylinder and directed away from the recording means; and a flash source of light to which the other end of the optical fibre is connected; characterised in that the hollow cylinder (24) does not contain any cylindrical lenses.

2. A photorefractometer according to Claim 1 in which the optical recording means is a photographic camera containing one or more spherical lenses.

3. An attachment for use in photorefractometry comprises a hollow cylinder; means to attach the cylinder to the front of a variable focus optical recording means containing a radially symmetrical focusing system, the cylinder having an internal diameter at least equal to the aperture of the camera, being attached coaxially with the focusing system, and containing an optical fibre arranged with one end coaxial with the cylinder and directed away from the recording means; and means to connect the other end of the fibre to a flash source of light; characterised in that the hollow cylinder does not contain any cylindrical lenses.

4. A method of testing an eye for defocus comprises illuminating the retina with a flash of light from a point source characterised by the steps of receiving light reflected by the retina at a position surrounding the point source and recording the received light through a radially symmetrical focusing system focused closer than the eye; repeating the illumination and recording the received light through a radially symmetrical focusing system focused beyond the eye; and from the two records determining the direction of any defocus of the eye.

5. A method of testing according to Claim 4 further comprising determining the axial orientation of any astigmatism of the eye.

0042240

- 10 -

6. A method of testing according to Claim 4 further comprising repeating the illumination and recording the received light through a radially symmetrical focusing system focused at the eye; and from the three records determining the magnitude of any defocus of the eye.

1/5

0042240

Fig. 1

Fig. 2

Fig. 3

Fig. 4

Fig. 5

Fig. 6

Fig. 7

0042240

Fig. 8

Fig. 9

| Category | DOCUMENTS CONSIDERED TO BE RELEVANT | | CLASSIFICATION OF THE APPLICATION (Int. Cl.) |
|---|---|---|---|
| | Citation of document with indication, where appropriate, of relevant passages | Relevant to claim | |
| AD | US - A - 3 879 113 (H.C. HOWLAND et al.) <br><br> * Abstract; column 1, line 39 - column 2, line 5; column 2, line 30 - column 3, line 67 and figures 1-4 * | 1,2,5 | A 61 B 3/14 <br> 3/00 <br> 3/02 |
| | FR - A - 2 387 020 (C. ZEISS) <br><br> * Page 1, line 13 - page 2, line 31 and figure 2 * | 1,2 | |
| | US - A - 3 903 870 (W.D. BERNDT/ APPLIED OPTICS CORP.) <br><br> * Abstract; column 2, line 30 - column 3, line 10; figures 1,2 * | 1,2 | **TECHNICAL FIELDS SEARCHED (Int. Cl.³)** <br><br> A 61 B 3/14 <br> 3/12 <br> 3/02 <br> 3/00 <br> G 02 B 5/16 |
| | DE - B - 1 217 652 (OPTISCHE WERKE G. RODENSTOCK) <br><br> * Column 1, line 46 - column 2, line 32; column 3, lines 1-46 * | 1,4,5 | |
| P | US - A - 4 259 948 (P. URBAN) <br><br> * Abstract; column 3, line 42 - column 5, line 33; figures 1-3 * | 1,2 | **CATEGORY OF CITED DOCUMENTS** <br><br> X: particularly relevant <br> A: technological background <br> O: non-written disclosure <br> P: intermediate document <br> T: theory or principle underlying the invention <br> E: conflicting application <br> D: document cited in the application <br> L: citation for other reasons |

The present search report has been drawn up for all claims

&: member of the same patent family, corresponding document

| Place of search | Date of completion of the search | Examiner |
|---|---|---|
| The Hague | 17-09-1981 | RIEB |

EPO Form 1503.1   06.78